# EUROPEAN PATENT SPECIFICATION

(11) **EP 0 508 940 B1**
(45) Date of publication and mention of the grant of the patent: **09.07.1997**
(21) Application number: 92810065.0
(22) Date of filing: 28.01.1992
(51) Int. Cl.: C07D 211/58

(54) **Process for preparing 2,2,6,6-tetra-methyl-4-piperidylamines**
Verfahren zur Herstellung von 2,2,6,6,-Tetramethyl-4-Amino-Piperidilen
Procédé pour la préparation de 2,2,6,6-tetraméthyl-4-piperidylamines

(30) Priority: 05.02.1991 IT MI910284
(43) Date of publication of application: 14.10.1992
(73) Proprietor: Ciba Specialty Chemicals Holding Inc., 4057 Basel (CH); Ciba Speciality Chemicals S.p.A., 40037 Sasso Marconi (BO) (IT)
(72) Inventor: Vignali, Graziano, I-40037 Sasso Marconi (Bologna) (IT); Guizzardi, Fabrizio, I-40128 Bologna (IT); Piccinelli, Piero, I-40037 Sasso Marconi (Bologna) (IT); Pedrini, Stefano, I-40128 Bologna (IT)

(56) References cited:
- EP-A- 0 033 529
- EP-A- 0 045 048
- EP-A- 0 061 785
- EP-A- 0 081 688
- EP-A- 0 208 455
- EP-A- 0 302 020
- EP-A- 0 354 184
- DE-A- 3 007 996
- US-A- 3 480 635

## Description

The present invention relates to a novel and convenient two-stage process for preparing some 2,2,6,6-tetramethyl-4-piperidylamines. The 2,2,6,6-tetramethyl-4-piperidylamines are compounds of considerable industrial interest, which can be used for preparing light stabilisers for synthetic polymers.

Their preparation has therefore been extensively described in the patent literature; it involves the reductive amination of 2,2,6,6-tetramethyl-4-piperidone in an organic solvent or without any solvent or in water or in a water/alcohols mixture in the presence of a hydrogenation catalyst such as platinum, palladium or nickel.

Representative publications are, in particular, the patents US-A-3,480,635, US-A-4,605,743 and EP-A-302,020.

The present invention relates to a two-stage process for preparing 2,2,6,6-tetramethyl-4-piperidylamines of the formula (I) 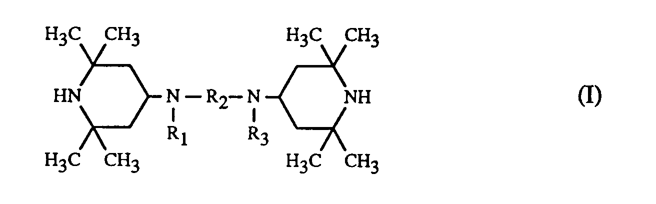 in which R₁ and R₃ independently of one another are hydrogen or methyl, R₂ is C₂-C₁₂alkylene, cyclohexylene, methylenedicyclohexylene, cyclohexylenedimethylene or C₄-C₁₂alkylene interrupted by 1, 2 or 3 oxygen atoms or by 1 or 2 >N-R₄ groups with R₄ being hydrogen or C₁-C₈alkyl, or the rest 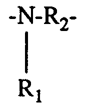 is a radical of the formula (IIa) or (IIb) 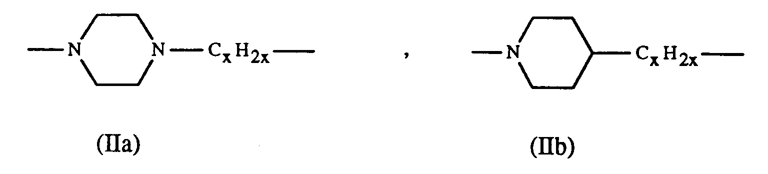 in which x is 1, 2 or 3, which comprises
(1) reacting 2,2,6,6-tetramethyl-4-piperidone with a mixture of (a) 80-100 % by weight of a compound of the formula (III) 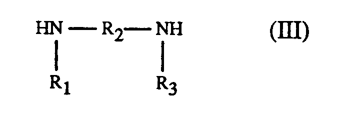 in which R₁, R₂ and R₃ are as defined above, and (b) 0-20 % by weight of water in the absence of a solvent at a temperature of 50-100°C, with simultaneously separating off the water of reaction and the component (b) by distillation at 1.3-180 mbar, and
(2) reacting the product obtained in the first stage with hydrogen in the presence of 0.001 to 0.04 % by weight of platinum or palladium or in the presence of 0.1 to 30 % by weight of Raney nickel, relative to the weight of the 2,2,6,6-tetramethyl-4-piperidone used in the first stage.

If R₁ and R₃ are hydrogen, the intermediate obtained in the first stage of the reaction corresponds to an imine of the formula (IVa)

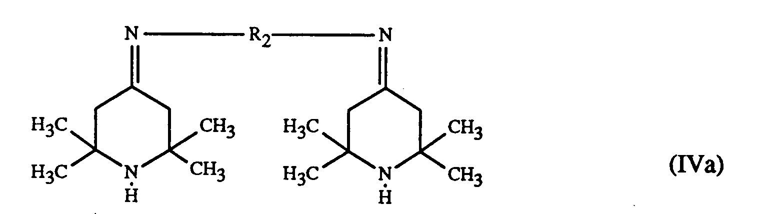

If R₁ and/or R₃ are other than hydrogen or the rest 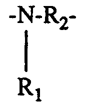 is a radical of the formula (IIa) or (IIb), the intermediate obtained in the first stage of the reaction corresponds to a compound of the formula (IVb) or (IVc)

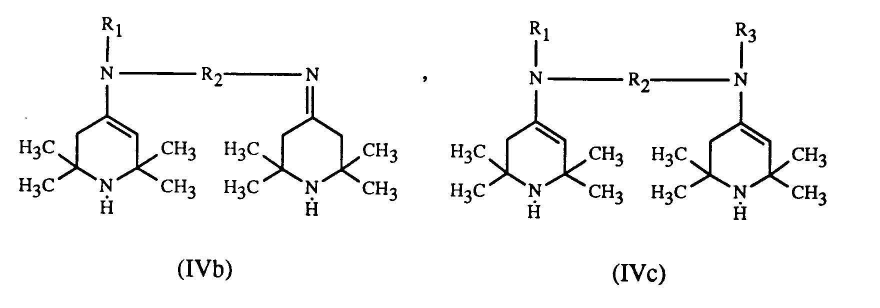

The process according to the present invention has various advantages over the state of the art:
I) in the first stage of the reaction, a quantitative yield of the intermediate of the formula (IVa), (IVb) or (IVc) is obtained, so that the quantity of unreacted 2,2,6,6-tetramethyl-4-piperidone is extremely small. Therefore, even the undesired and non-recyclable by-product 2,2,6,6-tetramethyl-4-piperidinol, normally present in the reaction mixture after the hydrogenation process, turns out to be absent in this case;
II) the almost complete elimination of water prevents the formation of various by-products, which are usually obtained at high temperatures (>80°C) during the hydrogenation process;
III) as a consequence of the elimination of water and of the undesired by-products, it is possible to raise the temperature of the reaction mixture in the second stage; in this way, the reaction time and the quantity of platinum or palladium can be considerably reduced. This is an important advantage, because the platinum and the palladium are poisoned during the hydrogenation and cannot be recycled. Moreover, because of the high temperature which can be reached in the second stage of the reaction, it is possible to use Raney nickel as the hydrogenation catalyst, which is less active but recyclable;
IV) the absence of flammable solvents eliminates the fire risk involved with the use of pyrophoric catalysts.

Representative examples of C₂-C₁₂alkylene R₂ are ethylene, propylene, trimethylene, tetramethylene, pentamethylene, 2,2-dimethyltrimethylene, hexamethylene, octamethylene, decamethylene and dodecamethylene. C₂-C₆Alkylene is preferred.

Representative examples of C₄-C₁₂akylene R₂ interrupted by 1, 2 or 3 oxygen atoms are 3-oxapentane-1,5-diyl, 4-oxaheptane-1,7-diyl, 3,6-dioxaoctane-1,8-diyl, 4,9-dioxadecane-1,12-diyl and 4,7,10-trioxatridecane-1,13-diyl.

Representative examples of C₄-C₁₂alkylene R₂ interrupted by 1 or 2 >N-R₄ groups are 3-azapentane-1,5-diyl, 3-azahexane-1,6-diyl, 4-azaheptane-1,7-diyl, 7-azatridecane-1,13-diyl, 4,7-diazadecane-1,10-diyl and 4-methyl-4-azaheptane-1,7-diyl.

Representative examples of C₁-C₈alkyl R₄ are methyl, ethyl, propyl, butyl, pentyl, hexyl, heptyl or octyl. Methyl, t-butyl and t-octyl are preferred.

Those compounds of the formula (I) are preferred in which R₁ and R₃ are hydrogen and R₂ is C₂-C₆alkylene, cyclohexylenedimethylene or C₄-C₁₀alkylene which is interrupted by 1 or 2 oxygen atoms or by an >N-R₄ group in which R₄ is hydrogen, methyl, t-butyl or t-octyl.

The molar ratio between the 2,2,6,6-tetramethyl-4-piperidone and the amine of the formula (III) can be the theoretical one.

It is also possible to use an excess of up to 20 % of piperidone over the theoretical.

Some diamines which can be used as reactants in the first stage of the reaction are commercially available as concentrated aqueous solutions; for example, hexamethylenediamine usually contains up to 15 % by weight of water.

Therefore apart from the anhydrous diamines, it is also possible to use a concentrated aqueous solution of diamines (mixture of components (a) and (b)).

A mixture comprising (a) 85-100 % by weight of the diamine of the formula (III) and (b) 0-15 % by weight of water is preferred as the starting material. The distillation, which is generally completed within 2-8 hours, is preferably carried out at a temperature of 60-80°C and a pressure of 10-130 mbar.

If desired, the pressure can be reduced at the end of the distillation down to 1.3 mbar, in order to eliminate all traces of unreacted 2,2,6,6-tetramethyl-4-piperidone.

If the hydrobenation catalyst used in the second stage is platinum or palladium, the reaction temperature is preferably 40-140°C; if the hydrobenation catalyst is Raney nickel, the reaction temperature is preferably 100-180°C.

The platinum and the palladium are preferably present in quantities of 0.001-0.02 % by weight and the Raney nickel is preferably present in quantities of 5-15 % by weight, relative to the weight of the 2,2,6,6-tetramethyl-4-piperidone used in the first stage.

The catalyst can be used unsupported or supported on suitable inert materials such as carbon, calcium carbonate, alumina and the like.

In general, the hydrogenation is carried out conveniently under a hydrogen pressure of 1-300 bar, preferably 1-100 bar, in particular 10-80 bar, and it is completed in approximately 2-4 hours.

After the hydrogenation, the catalyst can be separated off by filtration directly or after dilution with water.

The reaction products are conveniently purified by the usual procedures, for example by distillation or recrystallisation.

In general, the process according to the present invention is preferably carried out by introducing 2,2,6,6-tetramethyl-4-piperidone into a reactor at ambient temperature.

After the addition of component (a) and, if appropriate, (b) the reaction mixture is heated to the reaction temperature.

This normally requires half an hour to one hour, Subsequently, the water of reaction and the component (b) are distilled off in vacuo in the course of 2-8 hours. After the end of the first reaction stage, the catalyst is added and the reactor is flushed with an inert gas, for example nitrogen, and the reaction mixture is then hydrobenated at the reaction temperature.

The hydrogenation normally requires 2-8 hours depending on the hydrogen pressure, on the temperature and on the catalyst used.

The amines of the formula (III) are commercially available products or are easily obtainable by known processes, for example as described in US-A-3,513,170, US-A-3,959,295 and US-A-4,536,581.

To illustrate the present invention more clearly, several examples are given below.

### EXAMPLE 1: 257.5 g (1.659 mol) of 2,2,6,6-tetramethyl-4-piperidone and 102.3 g (0.806 mol) of hexamethylenediamine containing 8.5 % by weight of water are introduced into a 1 litre flask.

The mixture is then heated at 80°C for 1 hour with stirring, and the water of reaction and the water added then distilled off at 80°C and 40 mbar in the course of 3 hours.

The ketimine thus prepared is then placed into a 2 litre autoclave and 3.86 g of a mixture consisting of 1.93 g of 5 % platinum on carbon and 1.93 g of water are added.

The mixture is then hydrogenated at 90°C under a hydrogen pressure of 50 bar for 4 hours.

At the end of the reaction, the catalyst is removed by hot filtration and the N,N′-bis[2,2,6,6-tetramethyl-4-piperidyl]hexamethylenediamine is purified by distilling off the light ends (yield 97 %).

### EXAMPLE 2: 257.5 g (1.659 mol) of 2,2,6,6-tetramethyl-4-piperidone and 102.3 g (0.806 mol) of hexamethylenediamine containing 8.5 % by weight of water are introduced into a 1 litre flask.

The mixture is then heated at 80°C for 1 hour with stirring and the water of reaction and the water added then distilled off at 80°C and 30 mbar in the course of 5 hours.

The ketimine thus prepared is then placed into a 2 litre autoclave and 24 ml of decanted Raney nickel are added.

The mixture is then hydrogenated at 100°C and 100 bar for 4 hours.

At the end of the reaction, the catalyst is removed by hot filtration and the N,N′-bis(2,2,6,6-tetramethyl-4-piperidyl)hexamethylenediamine is purified by distilling off the light ends (yield 93 %).

### EXAMPLE 3: 341.5 g (2.2 mols) of 2,2,6,6,-tetramethyl-4-piperidone and 81.5 g (1.1 mol) of trimethylenediamine are introduced into a 1 litre flask.

The mixture is heated at 60°C for one hour with stirring and then the water of reaction is distilled off at 80°C and 40 mbar during 6 hours.

The ketimine thus prepared is placed into a 2 litres autoclave and 25 ml of decanted Raney nickel are added.

Subsequently, the mixture is hydrogenated at 100°C and 100 bar during 4 hours.

At the end of the reaction, the catalyst is removed by hot filtration and N,N′-bis(2,2,6,6-tetramethyl-4-piperidyl)trimethylenediamine is purified by distilling off the light ends (yield 95%).

## Claims

1. A 2-stage process for preparing a compound of the formula (I) 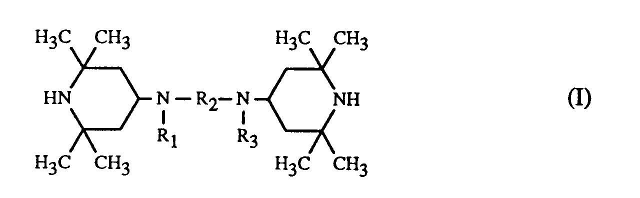 in which R₁ and R₃ independently of one another are hydrogen or methyl, R₂ is C₂-C₁₂alkylene, cyclohexylene, methylenedicyclohexylene, cyclohexylenedimethylene or C₄-C₁₂alkylene interrupted by 1, 2 or 3 oxygen atoms or by 1 or 2 >N-R₄ groups with R₄ being hydrogen or C₁-C₈alkyl, or the rest 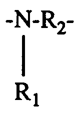 is a radical of the formula (IIa) or (IIb) 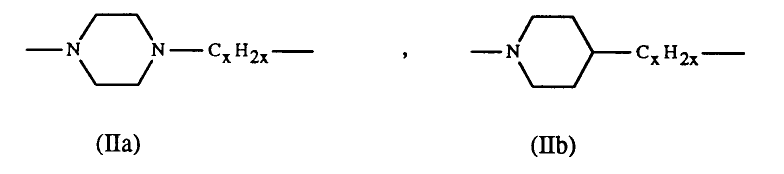 in which x is 1, 2 or 3, which comprises
(1) reacting 2,2,6,6-tetramethyl-4-piperidone with a mixture of (a) 80-100 % by weight of a compound of the formula (III) 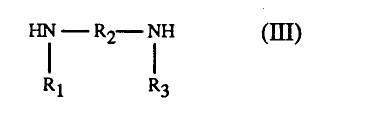 in which R₁, R₂ and R₃ are as defined above, and (b) 0-20 % by weight of water in the absence of a solvent at a temperature of 50-100°C, with simultaneously separating off the water of reaction and the component (b) by distillation at 1.3-180 mbar, and
(2) reacting the product obtained in the first stage with hydrogen in the presence of 0.001 to 0.04 % by weight of platinum or palladium or in the presence of 0.1 to 30 % by weight of Raney nickel, relative to the weight of the 2,2,6,6-tetramethyl-4-piperidone used in the first stage.

2. The process according to claim 1, wherein the molar ratio of 2,2,6,6-tetramethyl-4-piperidone and the compound of the formula (III) is between 2.0 and 2.4

3. The process according to claim 1, wherein the mixture comprises (a) 85-100 % by weight of the compound of the formula (III) and (b) 0-15 % by weight of water.

4. The process according to claim 1, wherein the distillation is carried out at a temperature of 60-80°C.

5. The process according to claim 1, wherein the distillation is carried out under a pressure of 10-130 mbar.

6. The process according to claim 1, wherein the distillation is carried out in the course of 2-8 hours.

7. The process according to claim 1, wherein the second stage is carried out in the presence of platinum or palladium as catalyst at a temperature of 40-140°C.

8. The process according to claim 1, wherein the second stage is carried out in the presence of Raney nickel as catalyst at a temperature of 100-180°C.

9. The process according to claim 1, wherein the platinum or palladium is used in a quantity of 0.001 to 0.02 % by weight.

10. The process according to claim 1, wherein the Raney nickel is used in a quantity of 5 to 15 % by weight.

11. The process according to claim 1, wherein the second stage is carried out under a hydrogen pressure of 1-300 bar.

12. The process according to claim 1, wherein the second stage is carried out in the course of 2-4 hours.

13. The process according to claim 1, wherein R₁ and R₃ are hydrogen and R₂ is C₂-C₆alkylene, cyclohexylenedimethylene, C₄-C₁₀alkylene interrupted by 1 or 2 oxygen atoms or by an >N-R₄ group with R₄ being hydrogen, methyl, t-butyl or t-octyl.

## Patentansprüche

1. Zweistufenverfahren zur Herstellung einer Verbindung der Formel (I) 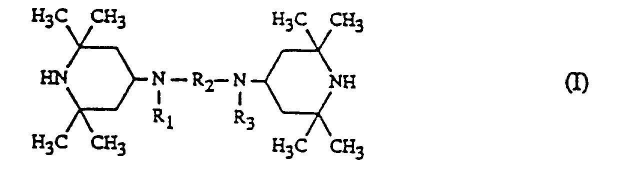 worin R₁ und R₃ unabhängig voneinander Wasserstoff oder Methyl darstellen, R₂ C₂-C₁₂-Alkylen, Cyclohexylen, Methylendicyclohexylen, Cyclohexylendimethylen oder C₄-C₁₂-Alkylen, unterbrochen durch 1, 2 oder 3 Sauerstoffatome oder durch 1 oder 2 Gruppen >N-R₄, wobei R₄ Wasserstoff oder C₁-C₈-Alkyl bedeutet, darstellt oder der Rest 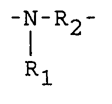 einen Rest der Formel (IIa) oder (IIb) 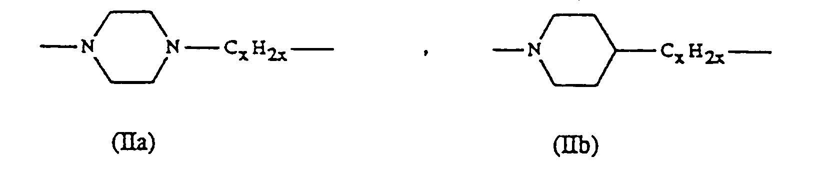 darstellt, worin x 1, 2 oder 3 ist, umfassend
(1) Umsetzen von 2,2,6,6-Tetramethyl-4-piperidon mit einen Gemisch von (a) 80-100 Gew.-% einer Verbindung der Formel (III) 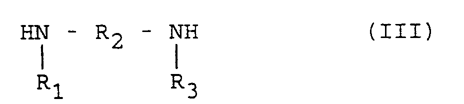 worin R₁, R₂ und R₃ wie vorstehend definiert sind und (b) 0-20 Gew.-% Wasser in Abwesenheit eines Lösungsmittels bei einer Temperatur von 50-100°C bei gleichzeitigem Abtrennen des Reaktionswassers und der Komponente (b) durch Destillation bei 1,3-180 mbar und
(2) Umsetzen des in der ersten Stufe erhaltenen Produkts mit Wasserstoff in Gegenwart von 0,001 bis 0,04 Gew.-% Platin oder Palladium oder in Gegenwart von 0,1 bis 30 Gew.-% Raney-Nickel, bezogen auf das Gewicht des 2,2,6,6-Tetramethyl-4-piperidons, das in der ersten Stufe verwendet wird.

2. Verfahren nach Anspruch 1, wobei das Molverhältnis von 2,2,6,6-Tetramethyl-4-piperidon und der Verbindung der Formel (III) zwischen 2,0 und 2,4 ist.

3. Verfahren nach Anspruch 1, wobei das Gemisch (a) 85-100 Gew.-% der Verbindung der Formel (III) und (b) 0-15 Gew.-% Wasser umfaßt.

4. Verfahren nach Anspruch 1, wobei die Destillation bei einer Temperatur von 60-80°C ausgeführt wird.

5. Verfahren nach Anspruch 1, wobei die Destillation unter einem Druck von 10-130 mbar ausgeführt wird.

6. Verfahren nach Anspruch 1, wobei die Destillation im Verlauf von 2-8 Stunden ausgeführt wird.

7. Verfahren nach Anspruch 1, wobei die zweite Stufe in Gegenwart von Platin oder Palladium als Katalysator bei einer Temperatur von 40-140°C ausgeführt wird.

8. Verfahren nach Anspruch 1, wobei die zweite Stufe in Gegenwart von Raney-Nickel als Katalysator bei einer Temperatur von 100-180°C ausgeführt wird.

9. Verfahren nach Anspruch 1, wobei Platin oder Palladium in einer Menge von 0,001 bis 0,02 Gew.-% verwendet wird.

10. Verfahren nach Anspruch 1, wobei das Raney-Nickel in einer Menge von 5 bis 15 Gew.-% verwendet wird.

11. Verfahren nach Anspruch 1, wobei die zweite Stufe unter einem Wasserstoffdruck von 1-300 bar ausgeführt wird.

12. Verfahren nach Anspruch 1, wobei die zweite Stufe im Verlauf von 2-4 Stunden ausgeführt wird.

13. Verfahren nach Anspruch 1, wobei R₁ und R₃ Wasserstoff darstellen und R₂ C₂-C₆-Alkylen, Cyclohexylendimethylen, C₄-C₁₀-Alkylen, unterbrochen durch 1 oder 2 Sauerstoffatome oder durch eine Gruppe >N-R₄, wobei R₄ Wasserstoff, Methyl, t-Butyl oder t-Octyl bedeutet, darstellt.

## Revendications

1. Procédé à 2 étapes pour préparer un composé de formule (I): 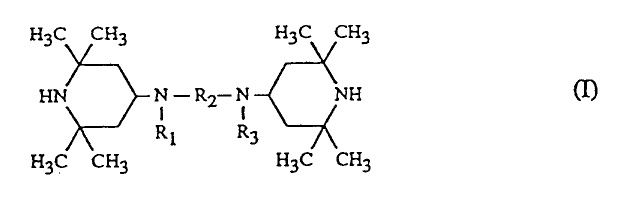 dans laquelle R₁ et R₃, indépendamment l'un de l'autre, sont un atome d'hydrogène ou un groupe méthyle, R₂ est un groupe alkylène en C₂ à C₁₂, cyclohexylène, méthylènedicyclohexylène, cyclohexylènediméthylène ou alkylène en C₄ à C₁₂ interrompu par 1, 2 ou 3 atomes d'oxygène ou par 1 ou 2 groupes >N-R₄, R₄ étant un atome d'hydrogène ou un groupe alkyle en C₁ à C₈, ou le reste 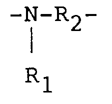 est un radical de formule (IIa) ou (IIb): 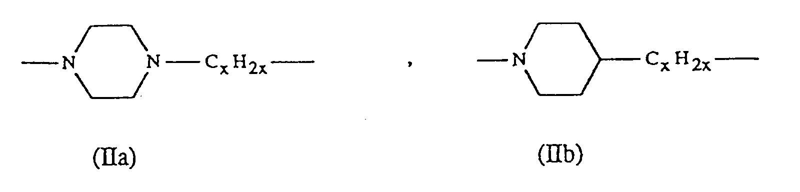 dans lesquelles x vaut 1, 2 ou 3, qui consiste:
(1) à faire réagir la 2,2,6,6-tétraméthyl-4-pipéridone avec un mélange (a) de 80 à 100% en poids d'un composé de formule (III): 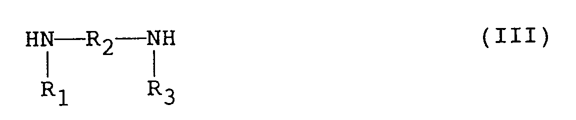 dans laquelle R₁, R₂ et R₃ sont tels que définis ci-dessus, et (b) de 0 à 20% en poids d'eau en l'absence d'un solvant à une température de 50 à 100°C, en séparant simultanément l'eau de la réaction et le composant (b) par distillation sous 1,3 à 180 mbar; et
(2) à faire réagir le produit obtenu dans la première étape avec de l'hydrogène en présence de 0,001 à 0,04% en poids de platine ou de palladium ou en présence de 0,1 à 30% en poids de nickel Raney, par rapport au poids de la 2,2,6,6-tétraméthyl-4-pipéridone utilisée dans la première étape.

2. Procédé selon la revendication 1, dans lequel le rapport molaire de 2,2,6,6-tétraméthyl-4-pipéridone et du composé de formule (III) est compris entre 2,0 et 2,4.

3. Procédé selon la revendication 1, dans lequel le mélange comprend (a) de 85 à 100% en poids du composé de formule (III) et (b) de 0 à 15% en poids en poids d'eau.

4. Procédé selon la revendication 1, dans lequel la distillation est effectuée a une température de 60 à 80°C.

5. Procédé selon la revendication 1, dans lequel la distillation est effectuée sous une pression de 10 à 130 mbar.

6. Procédé selon la revendication 1, dans lequel la distillation est effectuée au cours de 2 à 8 heures.

7. Procédé selon la revendication 1, dans lequel la deuxième étape est effectuée en présence de platine ou de palladium en tant que catalyseur à une température de 40 à 140°C.

8. Procédé selon la revendication 1, dans lequel la deuxième étape est effectuée en présence de nickel Raney en tant que catalyseur à une température de 100 à 180°C.

9. Procédé selon la revendication 1, dans lequel le platine ou le palladium est utilisé à raison de 0,001 à 0,02% en poids.

10. Procédé selon la revendication 1, dans lequel le nickel Raney est utilisé à raison de 5 à 15% en poids.

11. Procédé selon la revendication 1, dans lequel la deuxième étape est effectuée sous une pression d'hydrogène de 1 à 300 bar.

12. Procédé selon la revendication 1, dans lequel la deuxième étape est effectuée au cours de 2 à 4 heures.

13. Procédé selon la revendication 1, dans lequel R₁ et R₃ sont un atome d'hydrogène et R₂ est un groupe alkylène en C₂ à C₆, cyclohexylènediméthylène, alkylène en C₄ à C₁₀ interrompu par 1 ou 2 atomes d'oxygène ou par un groupe >N-R₄, R₄ étant un atome d'hydrogène, un groupe méthyle, t-butyle ou t-octyle.
